# FASCICULE DE BREVET EUROPEEN

(11) **EP 4 450 601 B1**
(45) Date de publication et mention de la délivrance du brevet: **15.10.2025**
(21) Numéro de dépôt: 24170998.9
(22) Date de dépôt: 18.04.2024
(51) Int. Cl.: C10L 3/10

(54) **INSTALLATION ET PROCEDE DE PRODUCTION DE METHANE PURIFIE**
ANLAGE UND VERFAHREN ZUR HERSTELLUNG VON GEREINIGTEM METHAN
PLANT AND PROCESS FOR PRODUCING PURIFIED METHANE

(30) Priorité: 20.04.2023 FR 2303953
(43) Date de publication de la demande: 23.10.2024
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: LOEB, Romain, 38360 SASSENAGE (FR); BERNHARDT, Jean-Marc, 38360 SASSENAGE (FR); ZICK, Golo, 38360 SASSENAGE (FR)
(74) Mandataire: Air Liquide

(56) Documents cités:
- FR-A1- 3 025 117
- FR-A1- 3 086 373

## Description

La présente invention est relative à une installation et un procédé de production de méthane purifié.

L'invention concerne plus particulièrement une installation de production de méthane purifié à partir de gaz naturel et de biogaz comprenant, disposés en série dans un circuit de gaz, une unité de retrait d'hydrocarbures lourds et une unité d'élimination du CO₂.

Le biogaz est le gaz produit lors de la dégradation de matières organiques en l'absence d'oxygène (fermentation anaérobie) encore appelée méthanisation. Il peut s'agir d'une dégradation naturelle que l'on observe dans les marais ou les décharges d'ordures ménagères. La production de biogaz peut aussi résulter de la méthanisation de déchets dans un réacteur dédié, et dont les conditions sont contrôlées. Ce réacteur est appelé méthaniseur ou digesteur, puis dans un post-digesteur, similaire au digesteur et permettant de pousser plus loin la réaction de méthanisation.

On appelle biomasse tout groupement de matières organiques pouvant se transformer en énergie à travers ce processus de méthanisation, par exemple boues de station d'épuration, fumiers/lisiers, résidus agricoles, et déchets alimentaires.

Le biogaz contient majoritairement du méthane (CH₄) et du dioxyde de carbone (CO₂) dans des proportions variables en fonction du mode d'obtention et du substrat mais peut également contenir, en moindres proportions de l'eau, de l'azote, de l'hydrogène sulfuré (H₂S), de l'oxygène, ainsi que des composés organiques autres, à l'état de traces, dont le H₂S, entre 10 et 50,000 ppmv.

Selon les matières organiques dégradées et les techniques utilisées, les proportions des composants diffèrent, mais en moyenne le biogaz comporte (en mole ou en volume) sur gaz sec, de 30 à 75% de méthane, de 15 à 60% de dioxyde de carbone, de 0 à 15% d'azote, de 0 à 5% d'oxygène et des composés sous forme de traces.

Le biogaz peut être valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.

Une purification plus poussée du biogaz permet sa plus large utilisation, en particulier, une purification poussée du biogaz permet d'obtenir un biogaz épuré aux spécifications du gaz naturel et qui pourra lui être substitué. Le biogaz ainsi purifié est appelé « biométhane ». Le biométhane complète ainsi les ressources de gaz naturel avec une partie renouvelable produite au cœur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (bio-GNL).

Les installations de liquéfaction pour produire du bio-GNL à partir de gaz naturel du réseau peuvent dans certains cas imposer une nouvelle configuration comprenant deux flux aux compositions, débits et pressions différentes (gaz naturel et biogaz).

Le gaz naturel est généralement distribué dans des réseaux hautes pressions (>40 barg) et possède des molécules impropres à la liquéfaction. On observe en général environ 1 à 2 mol% de CO₂ et de longues chaînes carbonées (hydrocarbures lourds). Le biogaz brut est lui produit à basse pression (>~ 0 barg) et avec une composition standard de 30 à 50 mol% de CO₂. Le reste étant très majoritairement composé de méthane et de quelques impuretés (H₂S...). Le biogaz brut généré est généralement amélioré en biométhane jusqu'à un taux de CO₂ de 2 à 4 mol% dans un process dit d'« upgrading » (séparation par membranes ou lavage aux amines) pour ensuite être injecté dans un réseau, liquéfié ou compressé en bouteille pour le transporter (CBG).

Pour produire du méthane à la qualité nécessaire requise à la liquéfaction, les solutions de lavage aux amines ou PTSA sont couramment utilisées pour épurer le flux de méthane comprenant 1 à 4 mol% CO₂ en entrée de ces unités à 50 à 400 ppm appelée « polishing ». Les hydrocarbures lourds doivent être évacués pour ne pas geler et boucher l'échangeur de chaleur principal d'une unité de liquéfaction de la même manière que le fait le CO₂ et donc arrêter le processus de liquéfaction. Les solutions de retrait des hydrocarbures lourds ne sont pas encore bien déterminées dans le marché pour les petites unités de liquéfaction tandis que pour les très grandes unités de liquéfaction (>100 tpd de gaz naturel) la solution onéreuse de distillation cryogénique peut être envisagée.

Pour produire du méthane purifié à la qualité ayant entre 0 et 400 ppm, en particulier entre 50 et 400 ppm, de CO₂ à partir de sources de gaz naturel et de biogaz, la solution traditionnelle est de mélanger les deux flux entrants. Le mélange se fait après le retrait des hydrocarbures lourds pour le flux de gaz naturel et après un « upgrading » standard (purification du biogaz brut pour produire du biométhane contenant 2 à 4% CO₂) du biogaz avec des membranes. Le mélange est effectué à la pression standard de liquéfaction du biogaz soit la pression atteignable en une seule unité de compression et nécessaire pour l'épuration membranaire (environ 12 barg).

Cette approche présente cependant les inconvénients suivants :
- Le gaz naturel doit être détendu de la pression du réseau à la pression du biométhane. Une perte d'énergie (énergie utilisable) conséquente serait donc constatée sur ce flux.
- L'opération d'« upgrading » produit un flux contenant un taux fixe de CO2 (le standard étant de 2 à 4 mol% en sortie).
- L'opération de purification supplémentaire, ou « polishing », nécessite un apport de chaleur pour l'élimination supplémentaire du CO₂, chaleur qui est fournie par une consommation d'énergie extérieure, par exemple par la combustion de gaz naturel (du réseau ou prélevé sur le débit entrant dans l'installation), par une source d'énergie électrique, ou par un mélange de plusieurs sources d'énergie. L'opération de purification supplémentaire peut par exemple être le lavage aux amines, pour purifier le méthane à la qualité nécessaire requise à la liquéfaction ou « polishing », par exemple ayant un taux de CO2 de 50 à 400 ppm.
- Lorsque l'« upgrading » est suivi par une opération de purification supplémentaire, celle-ci serait exécutée sur un flux contenant un taux fixe de CO2, qui ne serait pas optimisé, c'est à dire ni adapté ni adaptable, par rapport à la quantité d'énergie réellement disponible in situ pour l'opération de purification supplémentaire.
- L'« upgrading » du biogaz brut (la purification du biogaz brut) a un fort impact économique sur l'installation étant donné que les membranes ont un coût d'investissement élevé alors que de son côté le « polishing » secondaire (la purification du mélange de biogaz purifié et de gaz naturel pour atteindre un taux de CO₂ encore plus réduit), par exemple le lavage aux amines, nécessite un fort coût d'opération lié aux besoins en chaleur (proportionnel au taux de CO₂ à retirer).
- Un but de la présente invention est de pallier tout ou partie des inconvénients relevés ci-dessus.

L'art antérieur FR 3 086 373 propose pour sa part une installation et un procédé d'épuration et de liquéfaction de gaz naturel.

La présente invention propose une installation et un procédé innovants de production de méthane purifié provenant de deux sources différentes de gaz naturel et de biogaz qui permet d'optimiser l'opération de l'épuration du méthane. Cette optimisation permet d'utiliser la pression du réseau de gaz naturel avantageusement pour augmenter l'efficacité spécifique du liquéfacteur. Il est également possible d'optimiser l'unité de purification de biogaz en utilisant l'énergie disponible par les hydrocarbures lourds extraits du gaz naturel sur l'unité de retrait d'hydrocarbures lourds pour que l'énergie nécessaire pour une élimination supplémentaire du CO₂ soit égale à l'énergie obtenue en brûlant les hydrocarbures lourds.

L'installation selon l'invention, par ailleurs conforme à la définition générique qu'en donne le préambule ci-dessus, est essentiellement caractérisée en ce que l'unité de retrait d'hydrocarbures lourds comprend une première entrée destinée à être reliée à une source de gaz comprenant du gaz naturel, une première sortie de gaz épuré des hydrocarbures lourds, et une deuxième sortie d'hydrocarbures lourds retirés. L'unité d'élimination du CO₂ comprend une première entrée raccordée à la première sortie de l'unité de retrait d'hydrocarbures lourds, une première sortie de gaz à teneur réduite en CO₂, et une deuxième sortie de gaz effluents enrichi en CO₂. L'installation comprend en outre :
- une unité de purification de biogaz comprenant une entrée destinée à être reliée à une source de biogaz et une première sortie de biogaz purifié raccordée à la première entrée de l'unité d'élimination du CO₂, l'unité de purification de biogaz étant configurée pour produire du biogaz ou du biométhane avec une concentration variable/déterminée en CO₂, et
- une chaudière de production de chaleur par combustion d'hydrocarbure lourds comprenant une première entrée raccordée à la deuxième sortie de l'unité de retrait d'hydrocarbures lourds, la chaudière étant configurée pour produire une puissance thermique déterminée en fonction de la quantité d'hydrocarbures retirés et fournis par l'unité de retrait d'hydrocarbures lourds, la chaudière étant reliée fluidiquement à l'unité d'élimination du CO₂ pour fournir à cette dernière de l'énergie thermique produite pour l'élimination du CO₂.

Par ailleurs, des modes de réalisation de l'invention peuvent comporter l'une ou plusieurs des caractéristiques suivantes :
- l'unité d'élimination du CO₂ comprend ou est une unité de lavage aux amines,
- l'unité d'élimination du CO₂ est configurée pour avoir une capacité à retirer une quantité déterminée de CO₂ d'un flux gazeux, en particulier une capacité de lavage aux amines, ladite capacité étant fonction de l'énergie thermique fournie par la chaudière,
- l'installation est configurée pour mesurer ou déterminer l'énergie thermique produite par la chaudière et fournie à l'unité d'élimination du CO₂,
- l'installation est configurée pour moduler le niveau de purification du biogaz en fonction de l'énergie thermique produite par la chaudière et fournie à l'unité d'élimination du CO₂,
- la chaudière est configurée pour fournir à l'unité de retrait d'hydrocarbures lourds de l'énergie thermique,
- l'unité d'élimination du CO₂ comprend une troisième sortie de gaz comprenant un combustible et en ce que la chaudière comprend une deuxième entrée de gaz carburant raccordée à la troisième sortie de l'unité d'élimination du CO₂,
- l'unité de purification de biogaz est une unité de séparation par membrane,
- l'installation comporte une conduite destinée à relier la première sortie de biogaz purifié à un réseau de gaz naturel, et
- l'unité de purification de biogaz comprend une deuxième sortie de gaz riche en CO₂. L'invention concerne également un procédé de production de méthane purifié à partir de gaz naturel et de biogaz, le procédé comprenant :
- une étape d'alimentation en gaz comprenant du gaz naturel à une unité de retrait d'hydrocarbures lourds,
- une étape de retrait des hydrocarbures lourds dudit gaz par l'unité de retrait d'hydrocarbures lourds pour produire un gaz épuré en hydrocarbures lourds,
- une étape de fourniture du gaz épuré en hydrocarbures lourds à une unité d'élimination du CO₂, l'unité d'élimination du CO₂ assurant une élimination de CO₂ par un processus comprenant un chauffage,
- une étape d'alimentation d'une unité de purification de biogaz avec du biogaz,
- une étape de purification de biogaz dans l'unité de purification de biogaz pour produire un biogaz purifié,
- une étape de fourniture du biogaz purifié à l'unité d'élimination du CO₂,
- une étape de combustion, dans une chaudière, des hydrocarbures lourds retirés par l'unité de retrait d'hydrocarbures lourds,
- une étape de fourniture à l'unité d'élimination du CO₂ de l'énergie thermique produite par la combustion des hydrocarbures lourds,
- une étape d'élimination du CO₂ au sein du flux gazeux constitué par ou comprenant la somme du gaz épuré en hydrocarbures lourds et du biogaz purifié, l'étape d'élimination du CO₂ étant configurée pour réduire la concentration en CO₂ dans le flux gazeux à un seuil déterminé,
dans lequel l'étape de purification de biogaz est configurée pour produire un biogaz purifié ayant une concentration en CO₂ déterminée, ladite concentration étant choisie en fonction de la quantité totale de CO₂ à retirer du flux gazeux dans l'unité d'élimination du CO₂ pour atteindre le seuil de concentration de CO₂.

Selon d'autres particularités possibles :
- la quantité totale de CO₂ à retirer du flux gazeux dans l'unité d'élimination du CO₂ pour atteindre le seuil de concentration de CO₂ correspond à la capacité d'élimination du CO₂ de l'unité d'élimination, ladite capacité étant déterminée par l'énergie thermique produite et fournie par la chaudière,
- le procédé comprend une étape de détermination de l'énergie thermique produite par la chaudière et disponible pour l'unité d'élimination du CO₂, et
- le biogaz purifié est comprimé à la pression du gaz épuré des hydrocarbures lourds et combiné avec ce dernier pour constituer le flux gazeux fourni à l'unité d'élimination du CO₂.

L'invention peut concerner également tout dispositif ou procédé alternatif comprenant toute combinaison des caractéristiques ci-dessus ou ci-dessous dans le cadre des revendications. D'autres particularités et avantages apparaîtront à la lecture de la description ci-après, faite en référence à la figure.

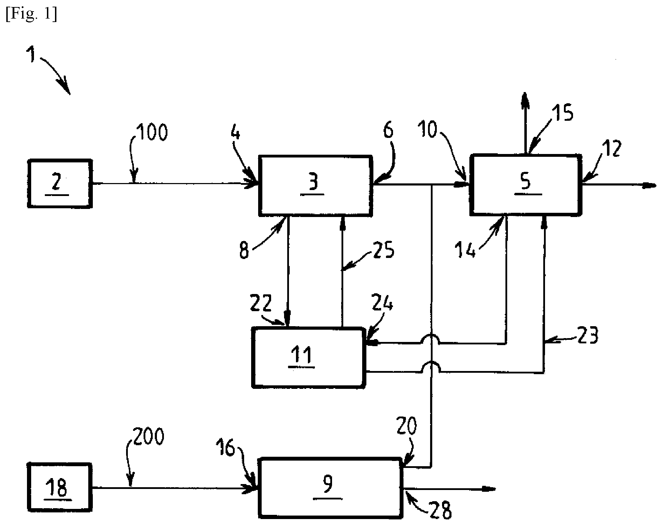

[Fig. 1] représente une vue schématique et partielle illustrant un exemple de réalisation de structure et de fonctionnement d'une installation selon l'invention.

L'exemple d'installation de production de méthane purifié illustré à la figure 1 comprend, disposées en série dans un circuit 100 de gaz naturel, une unité 3 de retrait d'hydrocarbures lourds et une unité 5 d'élimination du CO₂.

Dans un mode de réalisation, l'unité 3 de retrait d'hydrocarbures lourds comprend, par exemple, deux (ou plus) bouteilles d'adsorbant disposées en parallèles et fonctionnant de façon alternée. L'adsorbant comprend par exemple des filtres à charbon qui peuvent être régénérés par la chaleur. L'adsorbant adsorbe progressivement les espèces de carbone plus lourdes sur ses sites d'adsorption. Le gaz épuré, c'est-à-dire débarrassé des composés qui occupent les sites d'adsorption de l'adsorbant, circule en boucle fermée pour régénérer l'adsorbant sans pertes de méthane. Pendant la régénération de l'adsorbant d'une bouteille, une autre bouteille se met en opération de retrait d'hydrocarbures lourds. L'adsorbant est calibré pour piéger au moins une partie des hydrocarbures C3 (propane) et tous les hydrocarbures plus lourds (par exemple butane, pentane, hexane, aromatiques (benzène, éthylbenzène, xylène, toluène ...), entre autres).

L'unité 3 de retrait d'hydrocarbures lourds est par exemple une unité d'adsorption par variation de température et/ou de pression ou PTSA (Pressure-Temperature Swing Adsorption) avec 2 à 3 bouteilles d'adsorbant disposées en parallèles.

Dans un mode de réalisation, l'unité 3 de retrait d'hydrocarbures lourds peut comprendre au moins une colonne de distillation comprenant une solution de distillation des lourds par le froid.

La colonne de distillation retire un flux d'hydrocarbure à une température comprise entre -10 et -120 °C, en particulier entre -30 et -80 °C.

Les hydrocarbures retirés peuvent se présenter sous forme gazeuse ou liquide.

L'unité 3 de retrait d'hydrocarbures lourds comprend une première entrée 4 destinée à être reliée à une source de gaz 2 comprenant du gaz naturel (par exemple le gaz circulant dans un réseau local, régional ou national de distribution de gaz naturel). L'unité 3 de retrait d'hydrocarbures lourds comprend en outre une première sortie 6 de gaz épuré des hydrocarbures lourds, et une deuxième sortie 8 d'hydrocarbures lourds retirés. La source de gaz 2 est par exemple un réseau de gaz naturel sous pression.

L'unité 5 d'élimination du CO₂ comprend une première entrée 10 raccordée à la première sortie 6 de l'unité 3 de retrait d'hydrocarbures lourds, une première sortie 12 de gaz à teneur réduite en CO₂ et une deuxième sortie 15 de gaz effluents enrichi en CO₂.

L'installation 1 comprend une unité 9 de purification de biogaz qui est disposée en série avec l'unité 5 d'élimination du CO₂ dans un circuit 200 du biogaz.

L'unité 9 de purification de biogaz comprend une entrée 16 destinée à être reliée à une source 18 de biogaz brut, par exemple un digesteur, et une première sortie 20 de biogaz purifié, c'est-à-dire du biogaz présentant une concentration de CH₄ plus élevée et une concentration de CO₂ plus faible que le biogaz brut admis à l'entrée 16. Le biogaz purifié sortant de la première sortie 20 a un degré variable en CO₂, par exemple entre 2 et 20 %.

La première sortie 20 est raccordée à la première entrée 10 de l'unité 5 d'élimination du CO₂. L'unité 9 de purification de biogaz est configurée pour produire du biogaz épuré ou du biométhane avec une concentration variable/déterminée en CO₂.

L'unité 9 de purification de biogaz peut comprendre tout dispositif capable de produire du gaz enrichi en méthane à partir du biogaz brut, dont la puissance peut être régulée pour produire un gaz enrichi en méthane avec une concentration variable/déterminée de CO₂. L'unité 9 de purification de biogaz peut fonctionner selon le principe de la séparation et/ou de l'adsorption et/ou de l'absorption. Il peut s'agir par exemple, d'un système d'adsorption à pression alternée (PSA), de lavage aux amines, de lavage à l'eau, de lavage physique organique, de distillation cryogénique ou de séparation par membrane.

De préférence, l'unité 9 de purification de biogaz est une unité de traitement par perméation membranaire (ou une unité de séparation par membrane).

L'installation 1 comprend une chaudière 11 de production de chaleur par combustion d'hydrocarbures lourds. La chaudière 11 comprend une première entrée 22 raccordée à la deuxième sortie 8 de l'unité 3 de retrait d'hydrocarbures lourds. La première entrée 22 de la chaudière 11 est configurée pour alimenter la chaudière 11 en d'hydrocarbures lourds qui sortent via la deuxième sortie 8 de l'unité de 3 de retrait d'hydrocarbures lourds. La chaudière 11 est configurée pour produire une puissance thermique déterminée en fonction de la quantité d'hydrocarbures retirés et fournis par l'unité de retrait 3 d'hydrocarbures lourds. La chaudière 11 est reliée 23, par exemple au moyen d'un fluide caloporteur, à l'unité 5 d'élimination du CO₂ pour fournir à cette dernière de l'énergie thermique produite pour l'élimination du CO₂. L'unité 5 d'élimination du CO₂ est configurée pour avoir une capacité à retirer une quantité déterminée de CO₂ d'un flux gazeux qui est fonction de l'énergie thermique fournie par la chaudière 11.

L'énergie thermique produite par la combustion d'hydrocarbures lourds dans la chaudière 11 fournit l'énergie thermique nécessaire au fonctionnement de l'unité 5 d'élimination du CO₂. C'est-à-dire que la capacité d'élimination du CO₂ ou la quantité de CO₂ pouvant être retirée du flux gazeux introduit dans l'unité 5 dépend de la quantité d'hydrocarbures lourds retirée par l'unité de retrait 3 d'hydrocarbures lourds. La quantité totale de CO₂ contenue dans le flux total de gaz admis dans l'unité 5 d'élimination du CO₂ est la somme du CO₂ contenu dans le gaz provenant de la source de gaz 2 après passage dans l'unité de 3 de retrait d'hydrocarbures lourds et du CO₂ contenu dans le biogaz purifié sortant la sortie 20 de l'unité 9 de purification de biogaz.

Le gaz épuré en hydrocarbures lourds sort de l'unité de 3 de retrait d'hydrocarbures lourds par la première sortie 6 et est fourni à l'unité 5 d'élimination du CO₂ via la première entrée 10.

Avantageusement, la pression du gaz provenant de la source de gaz 2 reste constante, par exemple à 45 barg. Cette pression est maintenue élevée ou constante lors de toutes les étapes du procédé d'épuration (une étape de retrait des hydrocarbures lourds dudit gaz par l'unité 3 de retrait d'hydrocarbures lourds, une étape de fourniture du gaz épuré en hydrocarbures lourds à une unité 5 d'élimination du CO₂ et une étape d'élimination du CO₂).

En parallèle, l'unité 9 de purification de biogaz est alimentée avec du biogaz brut 18 via l'entrée 16 dans laquelle le biogaz est purifié pour produire un biogaz purifié ayant une concentration en CH₄ plus élevée et une concentration en CO₂ plus faible que le biogaz brut admis à l'entrée 16. Le biogaz purifié sortant la première sortie 20 est fourni à l'unité 5 d'élimination du CO₂ via la première entrée 10.

Le gaz épuré en hydrocarbures lourds et le biogaz purifié peuvent être alimentés à l'unité 5 d'élimination du CO₂ par la première entrée 10 de manière indépendante, c'est-à-dire que les deux flux de gaz sont combinés uniquement à l'intérieur de l'unité. Alternativement, et comme illustré, le gaz épuré en hydrocarbures lourds et le biogaz purifié peuvent être combinés en amont de l'unité 5 d'élimination du CO₂, par exemple par une liaison d'un conduit du gaz épuré en hydrocarbures lourds et d'un conduit du biogaz purifié, et alimenté à l'unité 5 en flux de gaz combiné.

Avantageusement, le biogaz purifié est comprimé à la pression du gaz provenant de la source de gaz 2, par exemple à 45 barg, avant d'être fourni à l'unité 5 d'élimination du CO₂ ou avant d'être combinés avec le gaz épuré en hydrocarbures lourds en amont de l'unité 5 d'élimination du CO₂.

L'installation 1 est configurée pour moduler le niveau de purification du biogaz en fonction de l'énergie thermique produite par la chaudière 11 et fournie à l'unité 5 d'élimination du CO₂.

Pour produire du méthane purifié ayant une concentration de CO₂ prédéterminée et sortant par la sortie 12 de l'unité 5 d'élimination du CO₂, la teneur total de CO₂ dans le flux gazeux admis à l'unité 5 est ajustée en fonction de l'énergie thermique disponible par la combustion des hydrocarbures lourds au sein de la chaudière 11. La teneur/quantité/concentration totale de CO₂ admis à l'unité 5 d'élimination du CO₂ est en particulier ajustée en faisant varier le degré de purification du biogaz brut dans l'unité 9 de purification du biogaz. Le degré de purification de biogaz conditionne le niveau de concentration en CO₂ dans le biogaz purifié sortant par la sortie 20 de l'unité 9 de purification de biogaz.

Le fonctionnement de l'unité 9 de purification de biogaz est optimisé en fonction de l'énergie disponible par la combustion des hydrocarbures lourds extraits sur l'unité 3 de retrait d'hydrocarbures lourds. L'installation 1 est configurée pour que l'énergie nécessaire au fonctionnement de l'unité 5 d'élimination du CO₂ dans le flux gazeux pour atteindre un seuil déterminé soit égale à l'énergie obtenue par la combustion des hydrocarbures lourds.

Le taux de CO₂ en sortie de l'unité 9 de purification de biogaz, c'est-à-dire la concentration molaire de CO₂ dans le biométhane, est déterminé par une fonction liée à un certain nombre de paramètres. Un unique optimum d'efficacité énergétique est trouvé en fonction des débits molaires du gaz comprenant le gaz naturel et du biogaz brut ainsi que des concentrations d' hydrocarbures lourds dans le gaz comprenant le gaz naturel et de CO₂ dans le biogaz brut. Ainsi, l'installation 1 selon l'invention permet de varier/moduler le degré d'épuration du biogaz afin d'optimiser l'efficacité énergétique et les coûts du matériel (« CAPEX ») liés à l'unité 9 de purification de biogaz.

Par exemple, l'unité 9 de purification de biogaz est une unité de traitement par perméation membranaire comprenant une ou plusieurs unités de séparation par membrane. Chaque unité de séparation par membrane (également appelée « étage ») peut comprendre une ou plusieurs membranes connectées en parallèle. Plusieurs étages de membrane (typiquement entre 2 et 4) sont nécessaires pour atteindre le taux d'épuration d'environ 2% de CO₂ en sortie de l'unité 9. Une épuration moins poussée permet de réduire ce nombre d'étage, par exemple de 1 à 2.

Dans un exemple de mise en œuvre de l'invention, l'unité 9 de purification du biogaz peut fonctionner dans un mode fixe dans lequel le niveau de purification du biogaz est prédéterminé/fixé. Ceci est applicable par exemple lorsque le débit et la composition du flux gazeux provenant de la source 2, en particulier la quantité d'hydrocarbures lourds fournis à la chaudière 11 est préalablement connue.

Dans un autre exemple de mise en œuvre de l'invention, l'installation peut être configurée pour moduler le niveau de purification du biogaz dans un mode dynamique en fonction de la quantité d'hydrocarbures lourds retirés par l'unité de retrait 3 d'hydrocarbures lourds ou de l'énergie thermique produite par la chaudière 11 fournie à l'unité 5 d'élimination du CO₂. L'installation 1 peut comprendre un dispositif de mesure ou d'analyse en continue de la quantité d'hydrocarbures lourds fournie à la chaudière 11 ou de l'énergie thermique produite par la chaudière 11 et fournie à l'unité 5 d'élimination du CO₂. L'installation 1 peut comprendre un organe pour envoyer un signal à l'unité 9 de purification de biogaz à partir de la valeur mesurée/analysée par le dispositif de mesure ou d'analyse. L'unité 9 de purification de biogaz peut être configurée pour recevoir ledit signal permettant d'ajuster le niveau de purification afin que la quantité de CO₂ restant dans le biométhane (biogaz traité) à la sortie de l'unité 9 de purification est régulée pour que la puissance thermique nécessaire à l'élimination du CO₂ à un seuil déterminé du gaz admis dans l'unité 5 d'élimination du CO₂ correspond à la puissance thermique générée par la combustion des hydrocarbures lourds dans la chaudière 11.

De préférence, l'unité 5 d'élimination du CO₂ comprend ou est une unité de lavage aux amines. Dans le cas où l'unité 5 d'élimination du CO₂ est une unité de lavage aux amines, la puissance thermique nécessaire à l'élimination du CO₂ correspond à la puissance thermique nécessaire à la régénération des amines. L'unité 5 d'élimination du CO₂ est ainsi configurée pour avoir une capacité de lavage aux amines en fonction de l'énergie thermique fournie par la chaudière 11.

Dans un exemple de mise en œuvre de l'invention, la chaudière 11 est configurée pour fournir à l'unité 3 de retrait d'hydrocarbures lourds de l'énergie thermique 25, par exemple au moyen d'un fluide caloporteur. L'énergie thermique produite par la combustion des hydrocarbures lourds dans la chaudière 11 peut être fournie en partie 25 à l'unité 3 de retrait d'hydrocarbures lourds et en partie 23 à l'unité 5 d'élimination du CO₂. L'énergie excédentaire après avoir fourni l'énergie nécessaire pour l'élimination 5 du CO₂ peut être fournie pour le retrait 3 au moins partiel des hydrocarbures lourds.

Comme visible à la figure 1, à cet effet, l'unité 5 d'élimination du CO₂ peut comprendre une troisième sortie 14 de gaz comprenant un combustible et la chaudière 11 peut comprendre une deuxième entrée 24 de gaz carburant raccordée à la troisième sortie 14 de l'unité 5 d'élimination du CO₂. L'unité 5 d'élimination du CO₂ (en particulier lorsque c'est une unité de lavage aux amines) peut être purgée (« flashed ») pour éliminer le CO₂ avec un flux comprenant des hydrocarbures résiduels. Ce gaz comprenant un combustible qui sort de la troisième sortie 14 de l'unité 5 d'élimination du CO₂ peut être fourni à la chaudière 11 par la deuxième entrée 24.

L'installation 1 peut comporter une conduite, par exemple une dérivation, destinée à relier également la première sortie 20 de biogaz purifié à un réseau de gaz naturel. Par exemple, il peut s'agir d'un réseau local, régional ou national de distribution de gaz naturel. Le biogaz purifié sortant de la première sortie 20 de l'unité 9 de purification de biogaz peut avoir une pureté correspondant à la spécification de biométhane suffisante pour être réinjecté dans le réseau de gaz naturel.

L'unité 9 de purification de biogaz peut comprendre une deuxième sortie 28 de gaz riche en CO2.

## Revendications

1. Installation (1) de production de méthane purifié à partir de gaz naturel (2) et de biogaz (18) comprenant, disposés en série dans un circuit (100) de gaz, une unité (3) de retrait d'hydrocarbures lourds et une unité (5) d'élimination du CO₂, l'unité de retrait (3) d'hydrocarbures lourds comprenant :
- une première entrée (4) destinée à être reliée à une source de gaz (2) comprenant du gaz naturel,
- une première sortie (6) de gaz épuré des hydrocarbures lourds, et
- une deuxième sortie (8) d'hydrocarbures lourds retirés,
l'unité (5) d'élimination du CO₂ comprenant :
- une première entrée (10) raccordée à la première sortie (6) de l'unité de retrait (3) d'hydrocarbures lourds,
- une première sortie (12) de gaz à teneur réduite en CO₂, et
- une deuxième sortie (15) de gaz effluents enrichi en CO₂,
l'installation (1) comprenant en outre :
- une unité (9) de purification de biogaz comprenant une entrée (16) destinée à être reliée à une source de biogaz (18) et une première sortie (20) de biogaz purifié raccordée à la première entrée (10) de l'unité (5) d'élimination du CO₂, l'unité (9) de purification de biogaz étant configurée pour produire du biogaz ou du biométhane avec une concentration variable/déterminée en CO₂, et
- une chaudière (11) de production de chaleur par combustion d'hydrocarbure lourds comprenant une première entrée (22) raccordée à la deuxième sortie (8) de l'unité (3) de retrait d'hydrocarbures lourds, la chaudière (11) étant configurée pour produire une puissance thermique déterminée en fonction de la quantité d'hydrocarbures retirés et fournis par l'unité de retrait (3) d'hydrocarbures lourds, la chaudière (11) étant reliée (23) fluidiquement à l'unité (5) d'élimination du CO₂ pour fournir à cette dernière de l'énergie thermique produite pour l'élimination du CO₂.

2. Installation (1) selon la revendication 1, **caractérisée en ce que** l'unité (5) d'élimination du CO₂ comprend ou est une unité de lavage aux amines.

3. Installation (1) selon la revendication 1 ou 2, **caractérisée en ce que** l'unité (5) d'élimination du CO₂ est configurée pour avoir une capacité à retirer une quantité déterminée de CO₂ d'un flux gazeux, en particulier une capacité de lavage aux amines, ladite capacité étant fonction de l'énergie thermique fournie par la chaudière (11).

4. Installation (1) selon l'une quelconque des revendications 1 à 3, **caractérisée en ce qu'**elle est configurée pour mesurer ou déterminer l'énergie thermique produite par la chaudière (11) et fournie à l'unité (5) d'élimination du CO₂.

5. Installation (1) selon l'une quelconque des revendications 1 à 4, **caractérisée en ce qu'**elle est configurée pour moduler le niveau de purification du biogaz en fonction de l'énergie thermique produite par la chaudière (11) et fournie à l'unité (5) d'élimination du CO₂.

6. Installation (1) selon l'une quelconque des revendications 1 à 5, **caractérisée en ce que** la chaudière (11) est configurée pour fournir à l'unité (3) de retrait d'hydrocarbures lourds de l'énergie thermique (25).

7. Installation (1) selon l'une quelconque des revendications 1 à 6, **caractérisée en ce que** l'unité (5) d'élimination du CO₂ comprend une troisième sortie (14) de gaz comprenant un combustible et **en ce que** la chaudière (11) comprend une deuxième entrée (24) de gaz carburant raccordée à la troisième sortie (14) de l'unité (5) d'élimination du CO₂.

8. Installation (1) selon l'une quelconque des revendications 1 à 7, **caractérisée en ce que** l'unité (9) de purification de biogaz est une unité de séparation par membrane.

9. Installation (1) selon l'une quelconque des revendications 1 à 8, **caractérisée en ce qu'**elle comporte une conduite destinée à relier la première sortie (20) de biogaz purifié à un réseau de gaz naturel.

10. Installation (1) selon l'une quelconque des revendications 1 à 9, **caractérisée en ce que** l'unité (9) de purification de biogaz comprend une deuxième sortie (28) de gaz riche en CO₂.

11. Procédé de production de méthane purifié à partir de gaz naturel et de biogaz, le procédé comprenant :
- une étape d'alimentation en gaz comprenant du gaz naturel (2) à une unité (3) de retrait d'hydrocarbures lourds,
- une étape de retrait des hydrocarbures lourds dudit gaz par l'unité (3) de retrait d'hydrocarbures lourds pour produire un gaz épuré en hydrocarbures lourds,
- une étape de fourniture du gaz épuré en hydrocarbures lourds à une unité (5) d'élimination du CO₂, l'unité (5) d'élimination du CO₂ assurant une élimination de CO₂ par un processus comprenant un chauffage,
- une étape d'alimentation d'une unité (9) de purification de biogaz avec du biogaz (18),
- une étape de purification de biogaz dans l'unité (9) de purification de biogaz pour produire un biogaz purifié,
- une étape de fourniture du biogaz purifié à l'unité (5) d'élimination du CO₂,
- une étape de combustion, dans une chaudière (11), des hydrocarbures lourds retirés par l'unité (3) de retrait d'hydrocarbures lourds,
- une étape de fourniture (23) à l'unité (5) d'élimination du CO₂ de l'énergie thermique produite par la combustion des hydrocarbures lourds,
- une étape d'élimination du CO₂ au sein du flux gazeux constitué par ou comprenant la somme du gaz épuré en hydrocarbures lourds et du biogaz purifié, l'étape d'élimination du CO₂ étant configurée pour réduire la concentration en CO₂ dans le flux gazeux à un seuil déterminé,
dans lequel l'étape de purification (9) de biogaz est configurée pour produire un biogaz purifié ayant une concentration en CO₂ déterminée, ladite concentration étant choisie en fonction de la quantité totale de CO₂ à retirer du flux gazeux dans l'unité (5) d'élimination du CO₂ pour atteindre le seuil de concentration de CO₂.

12. Procédé selon la revendication 11, **caractérisé en ce que** la quantité totale de CO₂ à retirer du flux gazeux dans l'unité (5) d'élimination du CO₂ pour atteindre le seuil de concentration de CO₂ correspond à la capacité d'élimination du CO₂ de l'unité (5) d'élimination, ladite capacité étant déterminée par l'énergie thermique produite et fournie par la chaudière (11).

13. Procédé selon la revendication 11 ou 12, **caractérisé en ce qu'**il comprend une étape de détermination de l'énergie thermique produite par la chaudière (11) et disponible pour l'unité (5) d'élimination du CO₂.

14. Procédé selon l'une quelconque des revendications 11 à 13, **caractérisé en ce que** le biogaz purifié est comprimé à la pression du gaz épuré des hydrocarbures lourds et combiné avec ce dernier pour constituer le flux gazeux fourni à l'unité (5) d'élimination du CO₂.

## Patentansprüche

1. Anlage (1) zur Herstellung von gereinigtem Methan aus Erdgas (2) und Biogas (18), umfassend, in Reihe in einem Gaskreislauf (100) angeordnet, eine Einheit (3) zur Entfernung von Schwerwasserstoffen und eine Einheit (5) zur Entfernung von CO2, wobei die Einheit (3) zur Entfernung von Schwerwasserstoffen umfasst:
• einen ersten Einlass (4), der dazu bestimmt ist, mit einer Gasquelle (2), die Erdgas umfasst, verbunden zu werden,
• einen ersten Auslass (6) für von Schwerwasserstoffen gereinigtes Gas, und
• einen zweiten Auslass (8) für entfernte Schwerwasserstoffe,
wobei die Einheit (5) zur Entfernung von CO2 umfasst:
• einen ersten Einlass (10), der an den ersten Auslass (6) der Einheit (3) zur Entfernung von Schwerwasserstoffen angeschlossen ist,
• einen ersten Auslass (12) für Gas mit reduziertem CO2-Gehalt, und
• einen zweiten Auslass (15) für mit CO2 angereichertes Abgas,
wobei die Anlage (1) ferner umfasst:
• eine Biogasreinigungsanlage (9), die einen Einlass (16), der dazu bestimmt ist, mit einer Biogasquelle (18) verbunden zu werden, und einen ersten Auslass (20) für gereinigtes Biogas umfasst, der an den ersten Einlass (10) der CO2-Entfernungseinheit (5) angeschlossen ist, wobei die Biogasreinigungsanlage (9) so konfiguriert ist, dass sie Biogas oder Biomethan mit einer variablen/festgelegten CO2-Konzentration produziert, und
• einen Heizkessel (11) zur Wärmeerzeugung durch Verbrennung von Schwerwasserstoffen, umfassend einen ersten Einlass (22), der an den zweiten Auslass (8) der Einheit (3) zur Entfernung von Schwerwasserstoffen angeschlossen ist, wobei der Heizkessel (11) so konfiguriert ist, dass er eine bestimmte thermische Leistung in Abhängigkeit von der Menge der von der Einheit (3) zur Entfernung von Schwerwasserstoffen entfernten und gelieferten Schwerwasserstoffe produziert, wobei der Heizkessel (11) fluidisch (23) mit der CO2-Entfernungseinheit (5) verbunden ist, um letztere mit der für die CO2-Entfernung erzeugten thermischen Energie zu versorgen.

2. Anlage (1) nach Anspruch 1, **dadurch gekennzeichnet, dass** die CO2-Entfernungseinheit (5) eine Aminwäscheeinheit umfasst oder ist.

3. Anlage (1) nach Anspruch 1 oder 2, **dadurch gekennzeichnet, dass** die CO2-Entfernungseinheit (5) so konfiguriert ist, dass sie eine bestimmte Menge CO2 aus einem Gasstrom entfernen kann, insbesondere eine Aminwäschekapazität, wobei diese Kapazität eine Funktion der vom Heizkessel (11) gelieferten thermischen Energie ist.

4. Anlage (1) nach einem der Ansprüche 1 bis 3, **dadurch gekennzeichnet, dass** sie so konfiguriert ist, dass sie die vom Heizkessel (11) erzeugte und an die CO2-Entfernungseinheit (5) gelieferte thermische Energie misst oder bestimmt.

5. Anlage (1) nach einem der Ansprüche 1 bis 4, **dadurch gekennzeichnet, dass** sie so konfiguriert ist, dass sie den Grad der Biogasreinigung in Abhängigkeit von der vom Heizkessel (11) erzeugten und an die CO2-Entfernungseinheit (5) gelieferten thermischen Energie moduliert.

6. Anlage (1) nach einem der Ansprüche 1 bis 5, **dadurch gekennzeichnet, dass** der Heizkessel (11) so konfiguriert ist, dass er die Einheit (3) zur Entfernung von Schwerwasserstoffen mit thermischer Energie (25) versorgt.

7. Anlage (1) nach einem der Ansprüche 1 bis 6, **dadurch gekennzeichnet, dass** die CO2-Entfernungseinheit (5) einen dritten Auslass (14) für ein brennbares Gas umfasst, und **dadurch gekennzeichnet, dass** der Heizkessel (11) einen zweiten Einlass (24) für Brenngas umfasst, der an den dritten Auslass (14) der CO2-Entfernungseinheit (5) angeschlossen ist.

8. Anlage (1) nach einem der Ansprüche 1 bis 7, **dadurch gekennzeichnet, dass** die Biogasreinigungsanlage (9) eine Membrantrennanlage ist.

9. Anlage (1) nach einem der Ansprüche 1 bis 8, **dadurch gekennzeichnet, dass** sie eine Leitung umfasst, die dazu bestimmt ist, den ersten Auslass (20) für gereinigtes Biogas mit einem Erdgasnetz zu verbinden.

10. Anlage (1) nach einem der Ansprüche 1 bis 9, **dadurch gekennzeichnet, dass** die Biogasreinigungsanlage (9) einen zweiten Auslass (28) für CO2-reiches Gas umfasst.

11. Verfahren zur Herstellung von gereinigtem Methan aus Erdgas und Biogas, wobei das Verfahren umfasst:
• einen Schritt der Zuführung von Gas, das Erdgas (2) umfasst, zu einer Einheit (3) zur Entfernung von Schwerwasserstoffen,
• einen Schritt der Entfernung der Schwerwasserstoffe aus diesem Gas durch die Einheit (3) zur Entfernung von Schwerwasserstoffen zur Herstellung von von Schwerwasserstoffen gereinigtem Gas,
• einen Schritt der Zuführung des von Schwerwasserstoffen gereinigten Gases zu einer Einheit (5) zur Entfernung von CO2, wobei die Einheit (5) zur Entfernung von CO2 eine CO2-Entfernung durch einen Prozess, der eine Heizung umfasst, gewährleistet,
• einen Schritt der Zuführung von Biogas (18) zu einer Biogasreinigungsanlage (9),
• einen Schritt der Reinigung von Biogas in der Biogasreinigungsanlage (9) zur Herstellung von gereinigtem Biogas,
• einen Schritt der Zuführung des gereinigten Biogases zu der CO2-Entfernungseinheit (5),
• einen Schritt der Verbrennung der von der Einheit (3) zur Entfernung von Schwerwasserstoffen entfernten Schwerwasserstoffe in einem Heizkessel (11),
• einen Schritt der Zuführung (23) der durch die Verbrennung der Schwerwasserstoffe erzeugten thermischen Energie zu der CO2-Entfernungseinheit (5),
• einen Schritt der Entfernung von CO2 innerhalb des Gasstroms, der die Summe des von Schwerwasserstoffen gereinigten Gases und des gereinigten Biogases bildet oder umfasst, wobei der CO2-Entfernungsschritt so konfiguriert ist, dass die CO2-Konzentration im Gasstrom auf einen bestimmten Schwellenwert reduziert wird,
wobei der Biogasreinigungsschritt (9) so konfiguriert ist, dass er gereinigtes Biogas mit einer bestimmten CO2-Konzentration produziert, wobei diese Konzentration in Abhängigkeit von der Gesamtmenge an CO2 gewählt wird, die aus dem Gasstrom in der CO2-Entfernungseinheit (5) entfernt werden muss, um den CO2-Konzentrationsschwellenwert zu erreichen.

12. Verfahren nach Anspruch 11, **dadurch gekennzeichnet, dass** die Gesamtmenge an CO2, die aus dem Gasstrom in der CO2-Entfernungseinheit (5) entfernt werden muss, um den CO2-Konzentrationsschwellenwert zu erreichen, der CO2-Entfernungskapazität der CO2-Entfernungseinheit (5) entspricht, wobei diese Kapazität durch die vom Heizkessel (11) erzeugte und gelieferte thermische Energie bestimmt wird.

13. Verfahren nach Anspruch 11 oder 12, **dadurch gekennzeichnet, dass** es einen Schritt zur Bestimmung der vom Heizkessel (11) erzeugten und für die CO2-Entfernungseinheit (5) verfügbaren thermischen Energie umfasst.

14. Verfahren nach einem der Ansprüche 11 bis 13, **dadurch gekennzeichnet, dass** das gereinigte Biogas auf den Druck des von Schwerwasserstoffen gereinigten Gases komprimiert und mit diesem kombiniert wird, um den der CO2-Entfernungseinheit (5) zugeführten Gasstrom zu bilden.

## Claims

1. Installation (1) for the production of purified methane from natural gas (2) and biogas (18) comprising, arranged in series in a gas circuit (100), a heavy hydrocarbon removal unit (3) and a CO2 removal unit (5), the heavy hydrocarbon removal unit (3) comprising:
• a first inlet (4) intended to be connected to a gas source (2) comprising natural gas,
• a first outlet (6) for gas purified from heavy hydrocarbons, and
• a second outlet (8) for removed heavy hydrocarbons,
the CO2 removal unit (5) comprising:
• a first inlet (10) connected to the first outlet (6) of the heavy hydrocarbon removal unit (3),
• a first outlet (12) for gas with reduced CO2 content, and
• a second outlet (15) for CO2-enriched effluent gas,
the installation (1) further comprising:
• a biogas purification unit (9) comprising an inlet (16) intended to be connected to a biogas source (18) and a first outlet (20) for purified biogas connected to the first inlet (10) of the CO2 removal unit (5), the biogas purification unit (9) being configured to produce biogas or biomethane with a variable/determined CO2 concentration, and
• a boiler (11) for heat production by combustion of heavy hydrocarbons comprising a first inlet (22) connected to the second outlet (8) of the heavy hydrocarbon removal unit (3), the boiler (11) being configured to produce a determined thermal power according to the quantity of heavy hydrocarbons removed and supplied by the heavy hydrocarbon removal unit (3), the boiler (11) being fluidically connected (23) to the CO2 removal unit (5) to supply the latter with the thermal energy produced for CO2 removal.

2. Installation (1) according to claim 1, **characterized in that** the CO2 removal unit (5) comprises or is an amine washing unit.

3. Installation (1) according to claim 1 or 2, **characterized in that** the CO2 removal unit (5) is configured to have a capacity to remove a determined quantity of CO2 from a gas flow, in particular an amine washing capacity, said capacity being a function of the thermal energy supplied by the boiler (11).

4. Installation (1) according to any one of claims 1 to 3, **characterized in that** it is configured to measure or determine the thermal energy produced by the boiler (11) and supplied to the CO2 removal unit (5).

5. Installation (1) according to any one of claims 1 to 4, **characterized in that** it is configured to modulate the level of biogas purification according to the thermal energy produced by the boiler (11) and supplied to the CO2 removal unit (5).

6. Installation (1) according to any one of claims 1 to 5, **characterized in that** the boiler (11) is configured to supply the heavy hydrocarbon removal unit (3) with thermal energy (25).

7. Installation (1) according to any one of claims 1 to 6, **characterized in that** the CO2 removal unit (5) comprises a third outlet (14) for gas comprising a combustible, and **in that** the boiler (11) comprises a second inlet (24) for fuel gas connected to the third outlet (14) of the CO2 removal unit (5).

8. Installation (1) according to any one of claims 1 to 7, **characterized in that** the biogas purification unit (9) is a membrane separation unit.

9. Installation (1) according to any one of claims 1 to 8, **characterized in that** it includes a line intended to connect the first outlet (20) for purified biogas to a natural gas network.

10. Installation (1) according to any one of claims 1 to 9, **characterized in that** the biogas purification unit (9) comprises a second outlet (28) for CO2-rich gas.

11. Method for the production of purified methane from natural gas and biogas, the method comprising:
• a step of feeding gas comprising natural gas (2) to a heavy hydrocarbon removal unit (3),
• a step of removing heavy hydrocarbons from said gas by the heavy hydrocarbon removal unit (3) to produce gas purified from heavy hydrocarbons,
• a step of supplying the gas purified from heavy hydrocarbons to a CO2 removal unit (5), the CO2 removal unit (5) ensuring CO2 removal by a process including heating,
• a step of feeding a biogas purification unit (9) with biogas (18),
• a step of purifying biogas in the biogas purification unit (9) to produce purified biogas,
• a step of supplying the purified biogas to the CO2 removal unit (5),
• a step of combustion, in a boiler (11), of the heavy hydrocarbons removed by the heavy hydrocarbon removal unit (3),
• a step of supplying (23) the CO2 removal unit (5) with the thermal energy produced by the combustion of heavy hydrocarbons,
• a step of removing CO2 from the gas flow constituted by or comprising the sum of the gas purified from heavy hydrocarbons and the purified biogas, the CO2 removal step being configured to reduce the CO2 concentration in the gas flow to a determined threshold,
wherein the biogas purification step (9) is configured to produce purified biogas having a determined CO2 concentration, said concentration being chosen according to the total quantity of CO2 to be removed from the gas flow in the CO2 removal unit (5) to reach the CO2 concentration threshold.

12. Method according to claim 11, **characterized in that** the total quantity of CO2 to be removed from the gas flow in the CO2 removal unit (5) to reach the CO2 concentration threshold corresponds to the CO2 removal capacity of the CO2 removal unit (5), said capacity being determined by the thermal energy produced and supplied by the boiler (11).

13. Method according to claim 11 or 12, **characterized in that** it includes a step of determining the thermal energy produced by the boiler (11) and available for the CO2 removal unit (5).

14. Method according to any one of claims 11 to 13, **characterized in that** the purified biogas is compressed to the pressure of the gas purified from heavy hydrocarbons and combined with the latter to constitute the gas flow supplied to the CO2 removal unit (5).
